Europäisches Patentamt

European Patent Office

Office européen des brevets

(19)

(11) **EP 0 930 843 B1**

(12) **EUROPÄISCHE PATENTSCHRIFT**

(45) Veröffentlichungstag und Bekanntmachung des Hinweises auf die Patenterteilung:
**25.02.2004 Patentblatt 2004/09**

(51) Int Cl.$^7$: **A61B 5/00**

(21) Anmeldenummer: **98928117.5**

(86) Internationale Anmeldenummer:
**PCT/DE1998/000937**

(22) Anmeldetag: **02.04.1998**

(87) Internationale Veröffentlichungsnummer:
**WO 1998/043534 (08.10.1998 Gazette 1998/40)**

(54) **VORRICHTUNG ZUR PHOTODYNAMISCHEN DIAGNOSE**

DEVICE FOR PHOTODYNAMIC DIAGNOSIS

DISPOSITIF DE DIAGNOSTIC PHOTODYNAMIQUE

(84) Benannte Vertragsstaaten:
**DE FR GB IT**

(30) Priorität: **02.04.1997 DE 19715320**
**22.05.1997 DE 19721454**

(43) Veröffentlichungstag der Anmeldung:
**28.07.1999 Patentblatt 1999/30**

(73) Patentinhaber: **Karl Storz GmbH & Co. KG**
**78532 Tuttlingen (DE)**

(72) Erfinder:
• **IRION, Klaus**
**D-78576 Emmingen-Liptingen (DE)**
• **STEPP, Herbert**
**D-81377 München (DE)**
• **EHRHARDT, André**
**D-78532 Tuttlingen (DE)**
• **TAFELMAIER, Hans**
**D-83026 Rosenheim (DE)**

(74) Vertreter: **Popp, Eugen, Dr.**
**Dr. Münich & Kollegen**
**c/o MEISSNER, BOLTE & PARTNER**
**Widenmayerstrasse 48**
**80538 München (DE)**

(56) Entgegenhaltungen:
**EP-A- 0 241 268**     **WO-A-97/11636**
**DE-A- 4 120 688**     **DE-A- 4 133 493**
**DE-A- 19 646 176**   **US-A- 4 056 724**

**Beschreibung**

**Technisches Gebiet**

Die Erfindung bezieht sich auf eine Vorrichtung zur "in vivo-Diagnose" mittels einer durch einen körpereigenen oder körperfremden Photosensibilisator lichtinduzierten Reaktion in biologischem Gewebe.

Um eine lichtinduzierte Reaktion in biologischen Systemen auszulösen, wird dem Patienten ein Photosensibilisator in einer Konzentration von wenigen mg/kg Körpergewicht verabreicht.

**Stand der Technik**

Typische Photosensibilisatoren sind Photofrin oder Photosan, die ein Hämatoporphyrin-Grundgerüst aufweisen, Delta-Aminolävulinsäure (ALA)-induziertes Protoporphyrin IX (PPIX), die seit kurzem in der Urologie und Dermatologie Verwendung findet, 9-OAc-Tetramethoxyethylporphycen, Benzoporphyrin-Derivate, Monoaspartyl-Chlorin $E_6$, m-Tetrahydroxyphenyl-Chlorin, Sn(IV)-Etiopurpurin oder Zn(II)-Phtalocyanin.

Diese Substanzen reichern sich in Tumorgeweben in ca. 2-bis 15-fach erhöhter Konzentration an. Diese selektive Anreicherung im Tumorgewebe stellt die entscheidende Grundlage für die photodynamische Diagnose (PDD) und die photodynamische Therapie (PDT) dar.

Zur Diagnose wird das zu untersuchende Gewebe nach geeigneter Wartezeit nach Verabreichung des Photosensibilisators mit blauem bzw. violettem Licht - bei bekannten Vorrichtungen nahezu ausschließlich Laserlicht - bestrahlt. Der Photosensibilisator, der im Tumorgewebe in einer erhöhten Konzentration vorliegt, wird durch dieses Licht angeregt und weist anschließend eine typische Rotfluoreszenz auf, durch die der Tumor lokalisiert werden kann.

Neben der Fluoreszenz - bewirkt durch einen im Gewebe angereicherten Photosensibilisator- kann auch die sogenannte Autofluoreszenz des Gewebes ausgelöst werden, die durch körpereigene Fluoreszenzfarbstoffe zustande kommt. Auch hier erfolgt die Anregung zumeist mit blauem bzw. UV-Licht.

Die photodynamische Diagnose (PDD) ist jedoch in Abhängigkeit von den verwendeten Photosensibilisatoren mit gewissen Problemen behaftet. Bei dem Einsatz von Photofrin und Photosan-3 als Photosensibilisatoren bei der photodynamischen Diagnose müssen für den Fluoreszenznachweis sehr aufwendige technische Vorrichtungen verwendet werden, da durch störende Eigenfluoreszenzanteile nur mit Hilfe sehr aufwendiger computergestützter Bildverarbeitungstechniken und hochempfindlichen Kameras mit Restlichtverstärker die Fluoreszenz des Tumorgewebes entsprechend nachgewiesen werden kann.

Bei der Verwendung von Delta-Aminolävulinsäure (ALA) ist die induzierte Fluoreszenz stark genug, daß sie rein visuell erkannt werden kann.

Aber auch die durch Delta-Aminolävulinsäure erreichte Fluoreszenz führt nicht zu einer optimalen Qualität des Bildes, das im Rahmen der Diagnose aufgezeichnet werden soll. Dies ist u.a. zurückzuführen auf Variabilitäten der optischen Gewebeparameter, die die Fluoreszenzintensität auf unspezifische Art und Weise beeinflussen.

Ferner ist bekannt, Photosensibilisatoren zur photodynamischen Therapie (PDT) einzusetzen. Hierzu wird auf die WO 93/20810 verwiesen, auf die im übrigen hinsichtlich der Erläuterung aller hier nicht näher beschriebenen Begriffe und Verfahrensschritte ausdrücklich Bezug genommen wird.

Die für die photodynamische Diagnose - auch als Fluoreszenz-Diagnose bezeichnet - bzw. für die photodynamische Therapie verwendeten Vorrichtungen, die auch als PDD- bzw. PDT-Vorrichtungen bezeichnet werden, weisen ein Beleuchtungssystem, eine lichtzuführende Einheit, die das Licht des Beleuchtungssystems auf den zu diagnostizierenden und/oder zu therapierenden Gewebebereich richtet, und eine bildgebende, eine bilderfassende sowie gegebenenfalls eine bildübertragende Einheit auf, die das von dem Gewebebereich kommende Licht in eine proximale Bildebene abbildet.

Beleuchtungssystem und lichtzuführende Einheit definieren den Beleuchtungsstrahlengang, während die bildgebende, die bilderfassende sowie gegebenenfalls die bildübertragende Einheit den Beobachtungsstrahlengang definieren.

Bei einer endoskopischen PDD-Vorrichtung besteht die lichtzuführende Einheit aus dem Lichtleiter, der das Beleuchtungssystem z.B. mit dem Lichtleiteranschluß des Endoskops verbindet, und dem Beleuchtungslichtleiter des Endoskops. Der Lichtleiter kann z.B. ein Quarzlichtleiter oder ein Fluidlichtleiter sein. Fluid- bzw. Quarzlichtleiter haben eine bessere Transmission im blauen bzw. violetten Bereich als Standard-Glaslichtleiter. Das distal angeordnete Objektiv des Endoskops, das den von dem aus dem Beleuchtungslichtleiter austretenden Licht beleuchteten Gewebebereich erfaßt, stellt die bilderfassende optische Einheit dar; das Bild des Objektivs wird beispielsweise mittels eines oder mehrerer CCD-Aufnehmer erfaßt, die als optoelektronische Bildwandlungs-Einheit dienen. Bei einer proximalen Anordnung der CCD-Aufnehmer wird das Bild des Objektivs zu den CCD-Aufnehmern von einem Relaislinsensystem oder einem Abbildungs-Faserbündel übertragen, die damit als bildübertragende Einheit dienen.

In der WO 97/11636 ist vorgeschlagen worden, eine endoskopische photodynamische Diagnose und Therapie mit einer Vorrichtung auszuführen, bei der als Lichtquelle nicht ein Laser, sondern eine "Weißlichtquelle" verwendet wird, also eine Lichtquelle, die inkohärentes Licht im Wellenlängenbereich wenigstens von 390 bis 650 nm erzeugt. Das Licht der Lichtquelle wird über eine fokussierende Einheit in das Lichtleitkabel eingekoppelt.

In dieser Anmeldung ist weiter vorgeschlagen worden, den spektralen Reintransmissionsgrad bzw. die (spektrale) Übertragungsfunktion der lichtzuführenden Einheit und den spektralen Reintransmissionsgrad bzw. die (spektrale) Übertragungsfunktion der bildgebenden bzw. bilderfassenden Einheit so aufeinander abzustimmen, daß nur ein derart bemessener Bruchteil des an dem bestrahlten Gewebe reflektierten Lichts zur Bilderzeugung beiträgt, daß das Fluoreszenzbild von diesem "Hintergrundbild" nicht überstrahlt wird.

Zur Einstellung der Übertragungsfunktion werden in der Regel Filtersysteme verwendet. Die bislang vorgeschlagenen Filtersysteme haben jedoch den Nachteil, daß bereits kleine toleranzbedingte Fehler insbesondere bei der Kantenlage und der Kantensteilheit zu großen Änderungen der zur Bilderzeugung beitragenden reflektierten Lichtmenge führen. Dies hat wiederum eine größere Änderung des Verhältnisses Fluoreszenzlicht zu Hintergrundlicht zur Folge.

Wird beispielsweise durch einen Fertigungs- oder Einbaufehler - Verkippung des Filters etc. - die Filterkurve des in den Beleuchtungsstrahlengang eingebrachten Filtersystems zu kürzeren Wellenlängen hin verschoben, verringert sich bereits bei kleinen Verschiebungen die Überlappung der Durchlaßbereiche der in den Beleuchtungsstrahlengang und in den Beobachtungsstrahlengang eingebrachten Filtersysteme praktisch auf "Null", so daß man kein Hintergrundbild aufgrund des direkt reflektierten Lichts und nur noch ein Fluoreszenzbild erhält.

Umgekehrt erhält man bereits bei einer kleinen Verschiebung zu längeren Wellenlängen hin eine zu große Überlappung, so daß das Fluoreszenzbild durch das sichtbare ("Nicht"-Fluoreszenz)-Hintergrundbild überstrahlt wird.

Weiterhin verschiebt eine Variation der Kantensteilheit auch die Lage der Transmissionskurve im unteren Transmisisonsbereich, so daß dieser Fehler wie ein Kantenlagefehler zu kompensieren ist. Im oberen Bereich verändert sich die Gesamttransmission allerdings nur geringfügig.

Ähnliche Probleme treten auch bei Vorrichtungen auf, bei denen eine photodynamische Diagnose mittels eines Mikroskops und insbesondere eines Operationsmikroskops ausgeführt wird. Entsprechende Vorrichtungen sind in der EP 0 241 268 A1 oder der US-PS 5,371,624 beschrieben.

Die Probleme, die bei der Filterauswahl auftreten können, sind auch in der US-PS 4,056,724 - vgl. insbesondere Fig. 14 - beschrieben.

Auf diese Druckschriften wird im übrigen zur Erläuterung aller hier nicht im einzelnen beschriebenen Begriffe ausdrücklich verwiesen.

## Darstellung der Erfindung

Der Erfindung liegt die Aufgabe zugrunde, eine spektrale Reintransmissionscharakteristik bzw. eine Übertragungsfunktion für den Beleuchtungsstrahlengang und/oder den Beobachtungsstrahlengang anzugeben, bei der sich toleranzbedingte Fehler insbesondere bei der Kantenlage und der Kantensteilheit deutlich geringer als bei anderen Systemen auf das Verhältnis der Lichtmengen des Fluoreszenzlichts und des direkt reflektierten und zur Bilderzeugung beitragenden Lichts auswirken.

Erfindungsgemäße Lösungen dieser Aufgabe sind im Patentanspruch 1 angegeben. Weiterbildungen der Erfindung sind Gegenstand der Ansprüche 2 bis 9.

Der Erfindung liegt der Grundgedanke zugrunde, die Transmissionskurven so zu gestalten, daß wenigstens eine der Transmissionskurven einen Bereich mit einer "flachen Steigung" aufweist, so daß Kantenlage- und Kantensteilheit-Fehler nur einen geringen Einfluß die Gesamttransmission haben. Hierdurch wird erreicht, daß die Gesamttransmission, die sich durch die Faltung der Transmission des Beleuchtungsstrahlungsgangs mit der Transmission des Beobachtungsstrahlungsgangs und Integration über den relevanten Wellenlängenbereich ergibt, nur geringfügig von der Verschiebung der Kantenlage und/oder der Kantensteilheit beeinflußt wird.

Erfindungsgemäß sind deshalb der spektrale Reintransmissionsgrad bzw. die spektrale Übertragungsfunktion $T_1(\lambda)$ der lichtzuführenden Einheit bzw. des Beleuchtungsstrahlengangs dem Fluoreszenzanregungsspektrum des Photosensibilisators bzw. des Gewebes und der spektrale Reintransmissionsgrad bzw. die spektrale Übertragungsfunktion $T_b(\lambda)$ der bildgebenden Einheit bzw. des Beobachtungsstrahlengangs dem Fluoreszenzspektrum des Photosensibilisators bzw. des Gewebes angepaßt. Weiterhin schneiden sich die Übertragungsfunktion $T_1(\lambda)$ des Beleuchtungsstrahlengangs und die Übertragungsfunktion $T_b(\lambda)$ des Beobachtungsstrahlengangs bei einem Transmissionswert von nicht mehr als 30%.

Die Erfindung geht von dem Grundgedanken aus, daß sich die Transmissionsgrade bzw. die spektralen Übertragungsfunktionen des Beleuchtungsstrahlenganges und des Beobachtungsstrahlenganges in einem Bereich schneiden, in dem wenigstens ein Transmissionsgrad eine - zumindest bei einer Mittelung bzw. einer Ersetzung der tatsächlichen Kurve durch eine Gerade über einen Wellenlängenbereich von 10-30 nm - flache Steigung hat, so daß durch eine Verschiebung einer oder beider Kurven sich der von den beiden Kurven eingeschlossene Bereich nur vergleichsweise wenig ändert.

Hierfür ist es entscheidend, daß es wenigstens eine Referenzwellenlänge $\lambda_r$ gibt, die höchstens $2\Delta\lambda$ größer oder kleiner als die Schnittpunktswellenlänge $\lambda_s$ ist, für die also gilt:

$$\lambda_s\text{-}2\Delta\lambda \leq \lambda_r \leq \lambda_s\text{+}2\Delta\lambda$$

und von der ausgehend

- die spektrale Übertragungsfunktion $T_1(\lambda)$ des Beleuchtungsstrahlengangs für wenigstens fünf Wellenlängen $\lambda_r$, $\lambda_r+\Delta\lambda$, $\lambda_r+3\Delta\lambda$, $\lambda_r-\Delta\lambda$ und $\lambda_r-2\Delta\lambda$ folgende Bedingungen erfüllt:

$$\left|T_1(\lambda_r\text{-}\Delta\lambda) - T_1(\lambda_r\text{-}2\Delta\lambda)\right| > 10\%$$

$$\left|T_1(\lambda_r\text{+}\Delta\lambda) - T_1(\lambda_r\text{+}3\Delta\lambda)\right| < 5\%, \text{ bevorzugt } <3\%$$

$$T_1(\lambda_r) > 0,5\ \%$$

$$T_1(\lambda_r\text{-}\Delta\lambda) > 0,5\ \%$$

$$T_1(\lambda_r\text{-}2\Delta\lambda) > 0,5\ \%$$

$$T_1(\lambda_r\text{+}\Delta\lambda) > 0,3\ \%$$

$$T_1(\lambda_r\text{+}3\Delta\lambda) > 0,3\ \%$$

mit

$$4\ nm < \Delta\lambda < 6\ nm,$$

- und die spektrale Übertragungsfunktion $T_b(\lambda)$ des Beobachtungsstrahlengangs für wenigstens fünf Wellenlängen $\lambda_r$, $\lambda_r-\Delta\lambda$, $\lambda_r-3\Delta\lambda$, $\lambda_r+\Delta\lambda$ und $\lambda_r+2\Delta\lambda$ folgende Bedingungen erfüllt:

$$\left|T_b(\lambda_r\text{+}\Delta\lambda) - T_b(\lambda_r\text{+}2\Delta\lambda)\right| > 10\%$$

$$\left|T_b(\lambda_r\text{-}\Delta\lambda) - T_b(\lambda_r\text{-}3\Delta\lambda)\right| < 5\%, \text{ bevorzugt } <3\%$$

$$T_b(\lambda_r) > 0,5\ \%$$

$$T_b(\lambda_r\text{+}\Delta\lambda) > 0,5\ \%$$

$$T_b(\lambda_r\text{+}2\Delta\lambda) > 0,5\%$$

$$T_b(\lambda_r\text{-}\Delta\lambda) > 0,3\ \%$$

$$T_b(\lambda_r - 3\Delta\lambda) > 0.3\ \%$$

mit

$$4\ nm < \Delta\lambda < 6\ nm.$$

Die Übertragungsfunktionen im lichtzuführenden und im bilderzeugenden Teil der erfindungsgemäßen Vorrichtung sind so gewählt, daß nur eine genau eingestellte Lichtmenge des direkt an dem Gewebe reflektierten Beleuchtungslichts, das naturgemäß eine vergleichsweise hohe Intensität hat, durch den bilderzeugenden Teil der Vorrichtung in die proximale Bildebene "gelangt", während Licht mit einer Wellenlänge $\lambda$, aus dem Bereich, in dem Fluoreszenz auftritt, nur dann in die proximale Bildebene gelangen kann, wenn es aus dem beleuchteten Gewebebereich und nicht aus dem Beleuchtungssystem kommt.

Dabei gewährleisten die erfindungsgemäß gewählten Übertragungsfunktionen des Beleuchtungsstrahlengangs und des Beobachtungsstrahlengangs der Vorrichtung, daß der beleuchtete Gewebebereich so stark mit Licht mit einer Wellenlänge beleuchtet wird, die nicht im Bereich im Bereich des Fluoreszenzspektrums liegt, daß die Untersuchungsperson aufgrund des in diesem Wellenlängenbereich direkt reflektierten Lichts, das ein Hintergrundbild liefert, Einzelheiten des beleuchteten Gewebebereichs unabhängig von der Fluoreszenzstrahlung wahrnehmen kann.

Anders ausgedrückt, wird erfindungsgemäß das Bild des mit Anregungslicht beleuchteten Gewebebereichs gleichzeitig mittels Fluoreszenzlicht und reflektiertem Beleuchtungslicht erzeugt, wobei die beiden zur Bilderzeugung beitragenden Anteile bezüglich ihrer Wellenlänge und bezüglich ihrer Intensität so eingestellt sind, daß sie sich nicht gegenseitig "stören".

Dabei ist es bevorzugt, wenn die Einstellung derart erfolgt, daß die Intensität des emittierten Fluoreszenzlichtes in der gleichen Größenordnung wie die Gesamtintensität des reflektierenden Anteils des Anregungslichtes des Beleuchtungssystems - gewichtet durch die Filtercharakteristik des Beobachtungssystems - liegt. Besonders vorteilhafter Weise erfolgt die Einstellung derart, daß die beiden Intensitäten in etwa gleich sind.

Weiterhin ist es von Vorteil, wenn sich die beiden spektralen Transmissionsgrade bei einem Wert von weniger als 10%, bevorzugt bei einem Wert von weniger als 5% schneiden (Anspruch 2).

Bei einer Weiterbildung der Erfindung weisen die Übertragungsfunktion des Beleuchtungsstrahlengangs im Bereich $\lambda_r...\lambda_r+3\Delta\lambda$ und/oder die Übertragungsfunktion des Beobachtungsstrahlengangs im Bereich $\lambda_r...\lambda_r-3\Delta\lambda$ ein nahezu horizontales Plateau oder ein lokales Maximum auf.

Wenn ALA-induziertes PPIX als Photosensibilisator gewählt wird, ist es bevorzugt, wenn der spektrale Transmissionsgrad des Beleuchtungsstrahlengangs die folgende Beziehung erfüllt

$$100\% > T_1(\lambda=400..420) \geq 80\%$$

$$15\% \geq T_1(\lambda=440..455) \geq 0.5\%.$$

Durch diese Ausbildung der spektralen Übertragungsfunktionen der lichtzuführenden Einheit und der bildgebenden Einheit wird erreicht, daß das Fluoreszenzlicht auf dem durch das Beleuchtungslicht erzeugten Bild beispielsweise in der Umgebung eines Tumors klar und kontrastreich wahrgenommen werden kann.

Zur Anpassung an die verschiedenen Photosensibilisatoren und/oder unterschiedliche diagnostische Bedingungen oder zur Umstellung der erfindungsgemäßen Vorrichtung auf ein therapeutisches Verfahren ist es weiterhin bevorzugt, wenn die Transmissionseigenschaften der lichtübertragenden und der bildgebenden Einheit mittels eines oder mehrerer optischer Elemente einstellbar sind.

Die optischen Elemente, die zur Einstellung der Übertragungsfunktionen der lichtübertragenden und der bildgebenden Einheit verwendet werden, sind bevorzugt Filtersysteme, wie z.B. Absorptionsfilter, Interferenzfilter oder auch Prismen sowie elektrisch ansteuerbare LC-Filter (liquid crystal filter), die in den Beleuchtungs- und den Beobachtungs-Strahlengang einbringbar sind. Dabei wird unter Beleuchtungsstrahlengang der Strahlengang von der Lampe der Lichtquelle zur lichtzuführenden Einheit, durch diese Einheit, und von dieser Einheit zum diagnostizierenden Gewebebereich verstanden. Die optischen Elemente und insbesondere die Filtersysteme können prinzipiell an jeder Stelle dieses Strahlengangs, bevorzugt an Stellen mit parallelem Strahlengang angeordnet sein. Besonders bevorzugt ist jedoch die Anordnung zwischen Beleuchtungssystem und lichtzuführender Einheit, also beispielsweise vor einem Lichtleiter-Faserbündel. Bei der Beschreibung des bzw. der Filtersysteme wird der Reintransmissionsgrad des jeweiligen Strahlengangs ohne Filtersystem als 100% angenommen.

Entsprechend wird unter Beobachtungs-Strahlengang der Strahlengang von dem beleuchteten Gewebebereich zur bildgebenden Einheit und von dieser zur proximalen Bildebene verstanden. (Ohne Filtersystem wird auch hier der Reintransmissionsgrad als 100% angenommen.) Eine Feinabstimmung der Transmissionsverläufe des Beleuchtungs- bzw. Beobachtungsstrahlengangs kann durch eine zusätzliche Verkippung der Filterelemente erfolgen.

Wenn die erfindungsgemäße Vorrichtung in ein Endoskop integriert ist, kann sich die Bildebene im Endoskop sowohl im Bereich des distalen Endes - beispielsweise bei Verwendung eines distal angeordneten Videochips - als auch im Bereich des proximalen Endes befinden. Im letzteren Falle weist der Beobachtungsstrahlengang neben einem Objektiv als bilderfassende optische Einheit beispielsweise ein Relaislinsensystem oder ein flexibles Faserbündel als bildübertragende Einheit auf. Bei Verwendung eines Relaislinsen-Systems oder eines Faserbündels als bildübertragende Einheit werden die in dem Beobachtungsstrahlengang eingebrachten Filtersysteme bevorzugt zwischen der "letzten Fläche" des Relaislinsen-Systems bzw. der Austrittsfläche des Faserbündels und der proximalen Bildebene angeordnet.

Bei Integration der erfindungsgemäßen Vorrichtung in ein Operationsmikroskop ist Bestandteil der bildgebenden Einheit das Mikroskop-Linsensystem, dem beispielsweise ein Videoaufnehmer als elektronisch bilderfassende Einheit nachgeordnet sein kann.

Die Farbfilter des Videochips gehen in die Filtercharakteristik nicht mit ein. Weitere eventuell im Strahlengang vorgesehene Filter sind jedoch bei der Bestimmung des Reintransmissionsgrades zu berücksichtigen.

Bei einer weiteren Ausgestaltung der Erfindung weist das in den Beleuchtungs-Strahlengang einbringbare Filter mindestens zwei getrennte Filter auf, von denen ein Filter eine thermostabile Interferenzfiltereinheit und das andere Filter ein thermostabiles Wärmeschutzfilter ist. Die thermostabile Interferenzfiltereinheit wiederum besteht bevorzugt aus einem Kurzpaß und einem Blockingfilter, die auf getrennten Substraten angeordnet sind. Hierdurch ergeben sich deutlich verbesserte Transmissionseigenschaften.

Die Verwendung von optischen Elementen und insbesondere von Filtern zur Beeinflussung der Strahlengang-Transmissionscharakteristik bzw. der Übertragungsfunktion hat den Vorteil, daß beispielsweise durch Ausschwenken der Filter eine normale Weißlicht-Beleuchtung und - Beobachtung erfolgen kann, so daß die Untersuchungsperson, also beispielsweise ein Arzt den mit Fluoreszenzdiagnose untersuchten Gewebebereich auch u.a. nach der Farbe beurteilen kann. Die Farbe ist beispielsweise im Bereich der Ophthalmologie ein wesentliches Beurteilungskriterium.

Als Lichtquellen können ebenfalls bekannte Lichtquellen und insbesondere bereits aus der Endoskopie bekannte Lichtquellen verwendet werden, die breitbandig in dem genannten Wellenlängenbereich Licht emittieren. Eine derartige Lichtquelle, die Licht in ausreichender Intensität emittiert, ist beispielsweise eine Gasentladungslampe und insbesondere eine Xenon-Gasentladungs-Hochdrucklampe. Sollte im Einzelfall die Lichtleistung der Lichtquelle nicht ausreichend sein, kann zusätzlich zu einer "kontinuierlich arbeitenden" Lichtquelle eine "gepulste" Lichtquelle, wie ein Blitzgerät mit Blitzlampe oder auch ein Laser eingesetzt werden.

## Kurze Beschreibung der Zeichnung

Die Erfindung wird nachstehend anhand von Ausführungsbeispielen unter Bezugnahme auf die Zeichnung näher beschrieben, in der zeigen:

Fig. 1      schematisch eine erfindungsgemäße Vorrichtung für endoskopische Anwendungen,
Fig. 2      schematisch die Filtercharakteristik eines Ausführungsbeispiels,
Fig. 3      vergrößert den Bereich, in dem sich die beiden Filterkurven schneiden, und
Fig. 4a     eine Darstellung zur Erläuterung der Vorteile der Erfindung, und
Fig. 4b     eine Darstellung zur Erläuterung der Nachteile des Standes der Technik

## Darstellung eines Ausführungsbeispiels

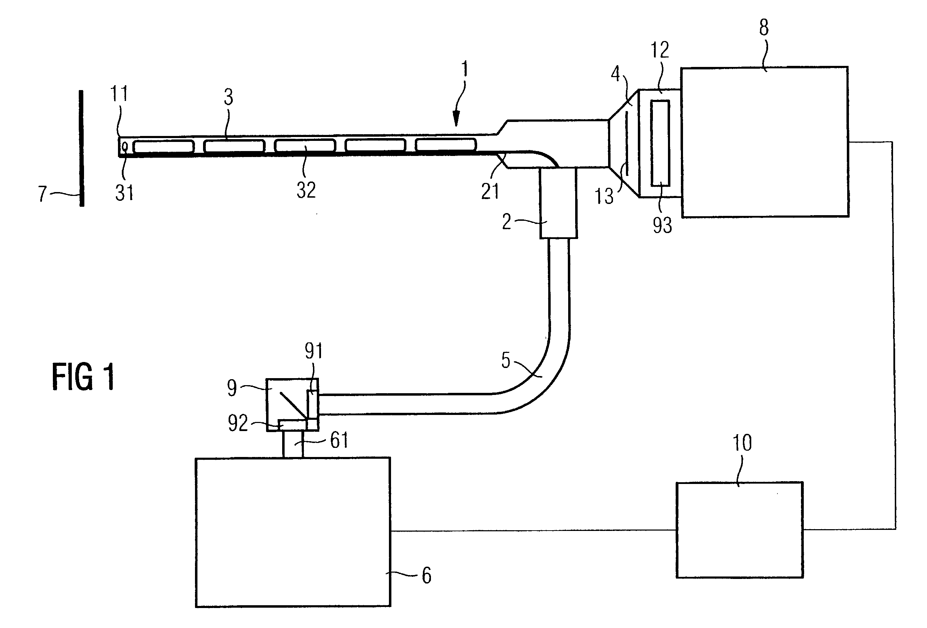

In Fig. 1 ist schematisch ein Ausführungsbeispiel einer erfindungsgemäßen Vorrichtung für endoskopische Anwendungen dargestellt. Das Bezugszeichen 1 bezeichnet ein Endoskop, das in bekannter Weise einen Lichtleiteranschluß 2, einen stabförmigen Teil 3, der in einen (nicht dargestellten) menschlichen Körper einsetzbar ist, und ein Okular 4 aufweist.

Der Lichtleiteranschluß 2 ist über ein Lichtleitkabel 5 mit einer Lichtquelle 6 verbundenen, die beispielsweise eine Xenon-Entladungslampe aufweisen kann. Ein z.B. aus einem Faserbündel bestehender Lichtleiter 21 in dem Endoskop 1 leitet das in den Lichtleiteranschluß 2 eingekoppelte Licht der Lichtquelle 6 zum distalen Ende 11 des Endoskops 1. Das aus dem distalen Ende 11 austretende Licht beleuchtet den zu untersuchenden Gewebebereich 7.

Das von dem Gewebebereich 7 kommende Licht tritt in ein nur schematisch dargestelltes Objektiv 31 des Endoskops 1 ein. Das Bild des Objektivs 31 wird durch einen Bildweiterleiter 32, der Stablinsen aufweisende Relaislinsen-Systeme oder ein Faser-Abbildungssystem aufweisen kann, zum proximalen Ende 12 des Endoskops 1 geleitet.

Das in der proximalen Bildebene 13 erzeugte Bild des Gewebebereichs 7 kann durch das Okular 4 mit dem Auge betrachtet werden. Alternativ oder über einen Strahlteiler zusätzlich zur Betrachtung mit dem Auge kann das Bild mit einer Videokamera 8 aufgenommen werden. In Fig. 1 ist die Alternative dargestellt, daß die Videokamera 8 direkt am Okular 4 angebracht ist.

Soweit wie vorstehend beschrieben ist der Aufbau beispielsweise durch mit einer Videokamera versehene Endoskope der Karl Storz GmbH & Co., Tuttlingen, Deutschland bekannt. Zum Detailaufbau wird deshalb auf die bekannten Endoskope dieses Herstellers Bezug genommen.

Zur Durchführung sogenannter photodynamischer Diagnosen können in den Beleuchtungsstrahlengang und in den Beobachtungsstrahlengang Filtersysteme eingebracht werden.

Hierzu ist bei dem in Fig. 1 dargestellten Ausführungsbeispiel an dem Licht-Ausgangsanschluß 61 der Lichtquelle 6 ein Filtersystem 9 angebracht, an dem wiederum das Lichtleitkabel 5 angeflanscht ist. Das Filtersystem 9 weist einen thermostabilen Interferenzfilter 91 und einen thermostabilen Wärmeschutzfilter 92 auf, der im wesentlichen die Wärmebeaufschlagung des Interferenzfilters 91 reduzieren soll. Auch vor der Videokamera 8 ist ein Filter 93 angebracht.

Die Belichtungseinstellung der Videokamera 8 und die Lichtabgabe der Lichtquelle 6 werden von einer Steuer- und Auswerteeinheit 10 gesteuert. Beispielsweise kann die Steuer- und Auswerteeinheit 10 eine Blitz-Lichtquelle mit der Lichtintegrationsphase eines CCD-Chips in der Videokamera 8 synchronisieren. Ferner kann die Steuer- und Auswerteeinheit 10 die von der Lichtquelle 6 abgegebene Lichtleistung und/oder die Belichtungseinstellung der Videokamera regeln.

An der Steuer- und Auswerteeinheit 10 liegt ferner das Ausgangssignal der Videokamera 8 an. Die Auswerteeinheit kann insbesondere ein Bildverarbeitungssystem aufweisen, das das Ausgangssignal der Videokamera in der einleitend beschriebenen Weise weiterverarbeitet und das bildverarbeitete Ausgangssignal auf einem Monitor darstellt. Selbstverständlich kann das unmittelbar von der Videokamera abgegebene und/oder das bildverarbeitete Ausgangssignal auch z.B. mittels eines Recorders gespeichert werden und/oder in einer Bilddatenbank abgelegt oder in sonstiger Weise mittels elektronischer Datenverarbeitung weiterverarbeitet werden.

Bei Verwendung eines Photosensibilisators geht von dem Gewebebereich 7 sowohl reflektiertes Beleuchtungslicht als auch Fluoreszenzlicht aus, das durch die von dem Photosensibilisator lichtinduzierte Reaktion in biologischen Systemen hervorgerufen wird. Um den verglichen mit dem reflektierten Licht geringen Anteil an Fluoreszenzlicht nachweisen und insbesondere bei einer nachfolgenden Bildverarbeitung sicher von dem "Nicht-Fluoreszenzlicht" trennen zu können, ist eine geeignet gewählte Transmissionscharakteristik des Beleuchtungs- und des Beobachtungsstrahlengangs erforderlich. Zur Einstellung der Transmissionscharakteristik während der photodynamischen Diagnose dienen die in den Strahlengang eingbringbaren Filter 91 und 93. Da die Filter beispielsweise durch Ausschwenken aus den Strahlengängen wieder entfernt werden können, ist auch eine normale Beobachtung des Gewebebereichs 7 möglich, ohne daß es beispielsweise zu einer Farbverfälschung kommen würde.

Im folgenden Ausführungsbeispiel wird exemplanish lediglich die Übertragungsfunktion des Beleuchtungsstrahlengangs diskutiert. In der beanspruchten Erfindung gemäß Anspruch 1 weisen Beleuchtungs- und Beobachtungsstrahlengang symmetrische Übertragungsfunktionen auf.

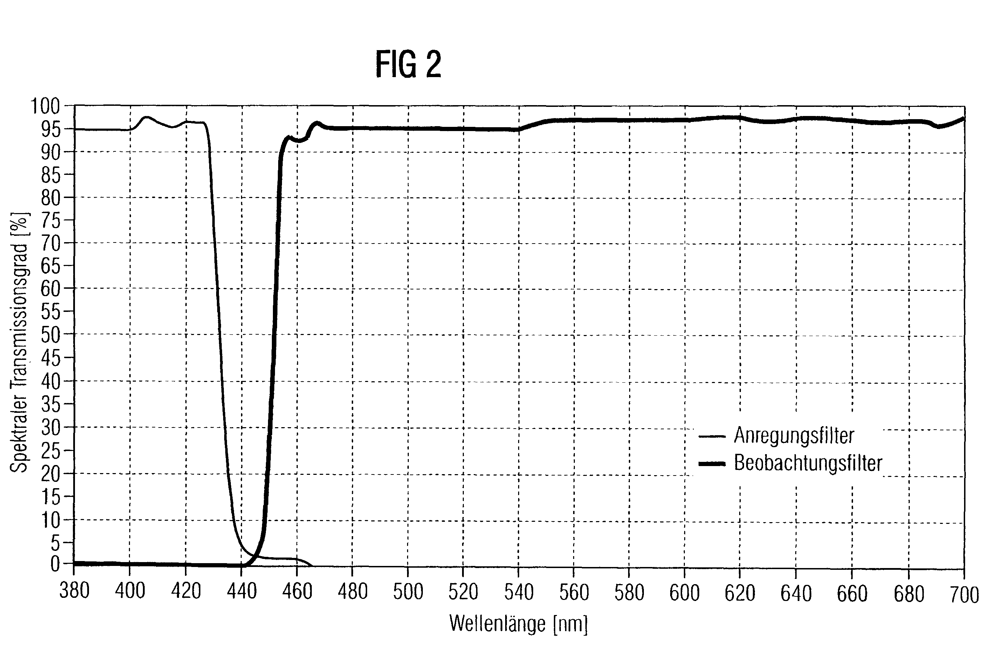

Nachfolgend soll unter Bezugnahme auf Fig. 2 die Charakteristik der Filter 91 und 93 erläutert werden. In den Figuren sind die Charakteristiken der Filter im Beleuchtungsstrahlengang (Anregungsfilter) und im Beobachtungsstrahlengang (Beobachtungsfilter) für ein Ausführungsbeispiel für den Fall angegeben, daß Delta-Aminolävulinsäure als Photosensibilisator verwendet wird. Bei Verwendung anderer Photosensibilisatoren ist die Filtercharakteristik entsprechend anzupassen.

Bezüglich der numerischen Werte der Durchlaßwerte bzw. des spektralen Transmissionsgrades $T(\lambda)$ (in %) als Funktion der Wellenlänge $\lambda$ wird ausdrücklich auf die Figuren verwiesen.

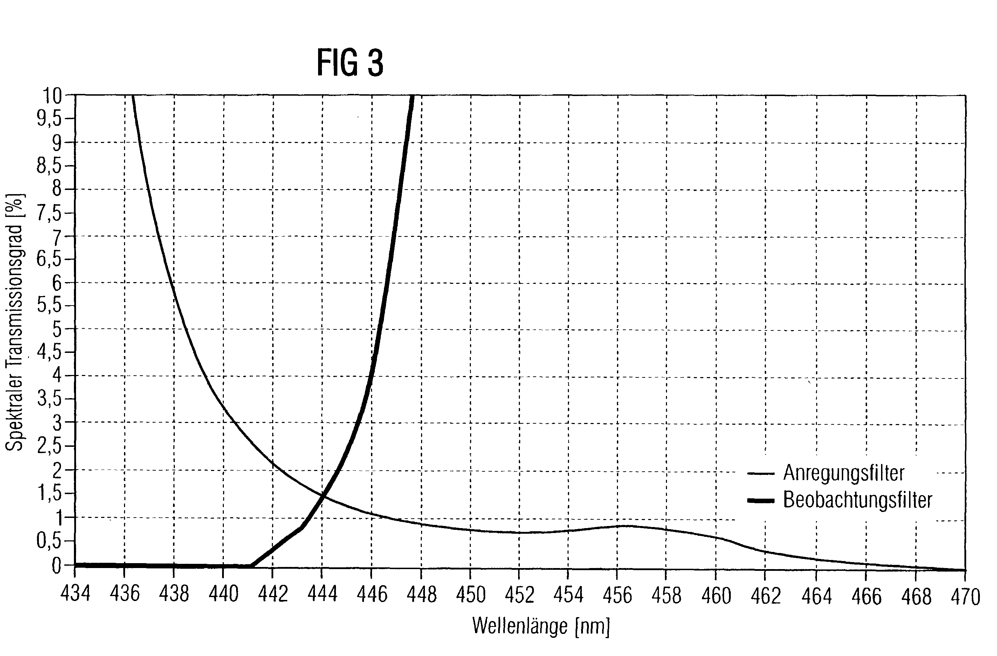

Fig. 2 ist zu entnehmen, daß die Transmission des Anregungsfilters ab etwa 425 nm einen steilen Abfall hin zu längeren Wellenlängen hat. Im Gegensatz zu der in Dokument WO97/11636 beschriebenen Filtercharakteristik ist die Transmission bei Wellenlängen, die größer als ca. 450 nm sind, jedoch nicht praktisch Null, sondern über einen Bereich von wenigstens 10 nm größer als 0,5 %, jedoch kleiner als 5%. (vgl. Fig. 3)

Dieser flache Auslauf über einen größeren Wellenlängenbereich der Transmissionskurve des Anregungsfilters bestimmt wesentlich die Überlappung der beiden Transmissionskurven, also die Menge des durchgelassenen Lichts, das vom Beobachter neben dem induzierten Fluoreszenzlicht als "Hintergrundbild" wahrgenommen wird.

Eine Verschiebung einer der beiden Kurven aufgrund von Fertigungsfehlern etc. hat damit einen wesentlich geringeren Einfluß auf die durchgelassene Lichtmenge als dies beim Stand der Technik der Fall ist.

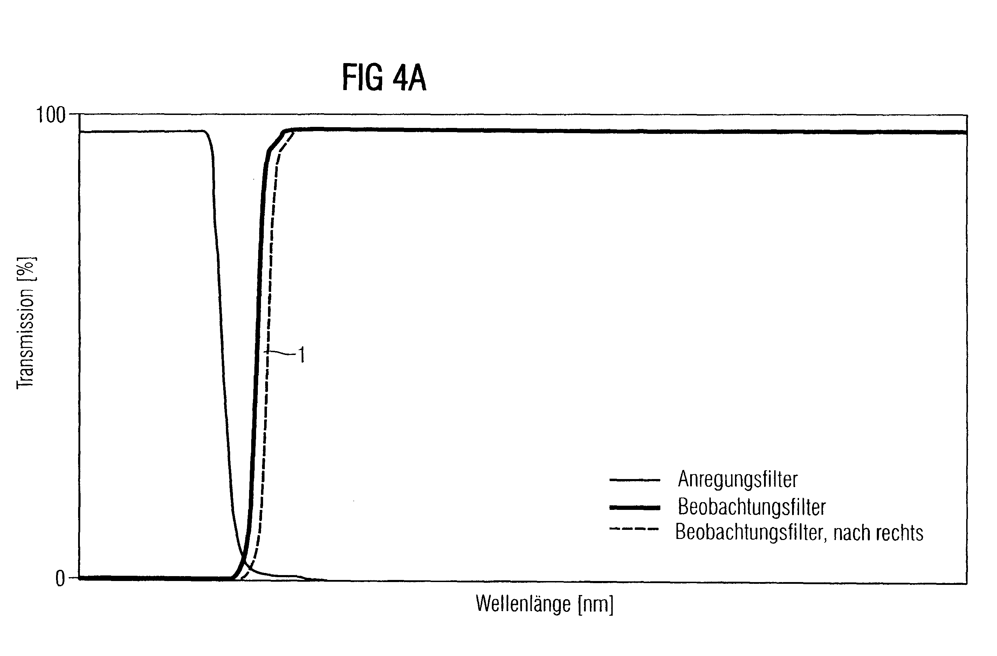

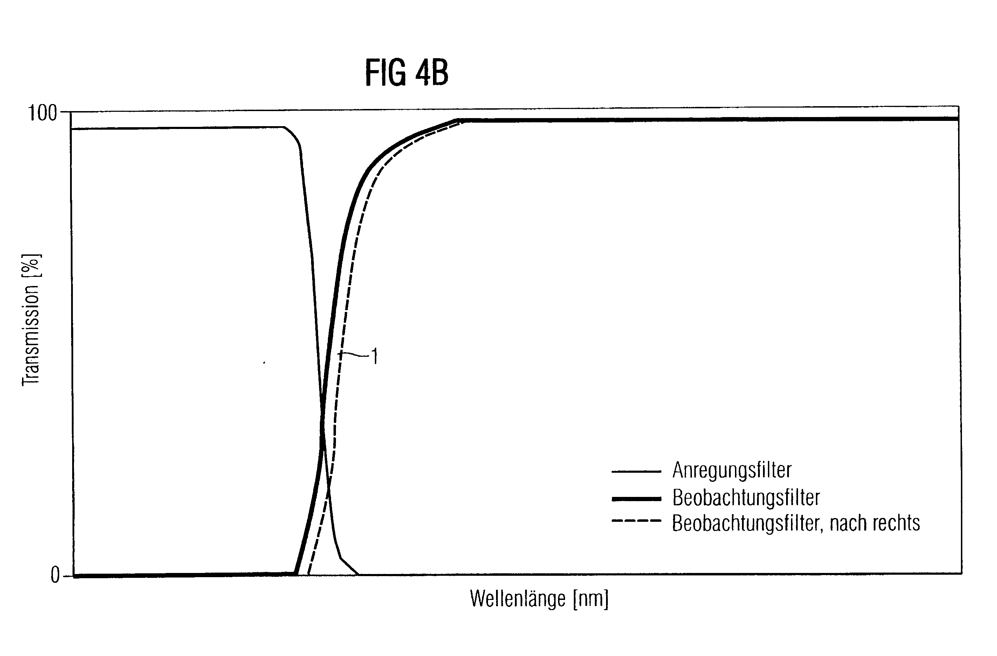

Dies zeigt Fig. 4a, in der zusätzlich zur Soll-Charakteristik des Beobachtungsfilters eine durch Fertigungsfehler hervorgerufene "Ist-Charakteristik" gestrichelt angegeben ist, bei der die Filterkurve um eine bestimmte Wellenlänge A verschoben ist. Wie man aus Fig. 4b entnimmt, wirkt sich der Fehler in der Charakteristik nur geringfügig auf die durchgelassene Lichtmenge aus.

Fig. 4b zeigt zum Vergleich die Änderung beim Stand der Technik, bei dem die Transmission des Anregungsfilters kein erfindungsgemäßes Plateau aufweist, sondern direkt auf Null abfällt: Durch die Verschiebung des Transmission um den Wert $\Lambda$ wird die Menge des Lichts, das zum Hintergrundbild beiträgt, wesentlich mehr als bei der Erfindung verringert.

## Patentansprüche

1. Endoskopische oder mikroskopische Vorrichtung zur Diagnose mittels einer durch einen Photosensibilisator lichtinduzierten oder durch Eigenfluoreszenz hervorgerufenen Reaktion in biologischem Gewebe "in vivo", mit

   - einem Beleuchtungsstrahlengang, gebildet von

     einem Beleuchtungssystem, das mindestens eine Lichtquelle (6) mit einem Lampensystem aufweist, das inkohärentes Licht in einem Wellenlängenbereich wenigstens von 400 bis 650 nm erzeugt, und
     einer lichtzuführenden Einheit (9,5), die das Licht des Beleuchtungssystems auf den zu diagnostizierenden und/oder zu therapierenden Gewebebereich richtet,

     der eine spektrale Übertragungsfunktion $T_1(\lambda)$ aufweist, die dem Fluoreszenzanregungsspektrum des Photosensibilisators bzw. des Gewebes angepaßt ist, und
   - einem Beobachtungsstrahlengang, gebildet von

     einer bildgebenden (4), einer bilderfassenden (8) sowie gegebenenfalls einer bildübertragenden Einheit, die das von dem Gewebebereich kommende Licht in eine Bildebene abbildet,

     der eine spektrale Übertragungsfunktion $T_b(\lambda)$ aufweist, die dem Fluoreszenzspektrum des Photosensibilisators bzw. des Gewebes angepaßt ist,

   wobei die spektrale Übertragungsfunktion $T_1(\lambda)$ des Beleuchtungsstrahlengangs und die spektrale Übertragungsfunktion $T_b(\lambda)$ des Beobachtungsstrahlengangs sich bei einer Wellenlänge $\lambda_s$ schneiden, bei der der Transmissionswert jedes Strahlengangs nicht mehr als 30% beträgt,
   **dadurch gekennzeichnet, daß** es wenigstens eine Referenzwellenlänge $\lambda_r$ gibt, die höchstens $2\Delta\lambda$ größer oder kleiner als die Schnittpunktswellenlänge $\lambda_s$ ist, für die also gilt:

$$\lambda_s\text{-}2\Delta\lambda < \lambda_r < \lambda_s\text{+}2\Delta\lambda$$

   und von der ausgehend

   - die spektrale Übertragungsfunktion $T_1(\lambda)$ des Beleuchtungsstrahlengangs für wenigstens fünf Wellenlängen $\lambda_r$, $\lambda_r\text{+}\Delta\lambda$, $\lambda_r\text{+}3\Delta\lambda$, $\lambda_r\text{-}\Delta\lambda$ und $\lambda_r\text{-}2\Delta\lambda$ folgende Bedingungen erfüllt:

$$\left|T_1(\lambda_r\text{-}\Delta\lambda) \text{ - } T_1(\lambda_r\text{-}2\Delta\lambda)\right| > 10\%$$

$$\left|T_1(\lambda_r\text{+}\Delta\lambda) \text{ - } T_1(\lambda_r\text{+}3\Delta\lambda)\right| < 5\%, \text{ bevorzugt } <3\%$$

$$T_1(\lambda_r) > 0,5\ \%$$

$$T_1(\lambda_r\text{-}\Delta\lambda) > 0,5\ \%$$

$$T_1(\lambda_r\text{-}2\Delta\lambda) > 0,5\ \%$$

$$T_1(\lambda_r\text{+}\Delta\lambda) > 0,3\ \%$$

$$T_1(\lambda_r+3\Delta\lambda) > 0{,}3\ \%$$

mit

$$4\ nm < \Delta\lambda < 6\ nm,$$

- und die spektrale Übertragungsfunktion $T_b(\lambda)$ des Beobachtungsstrahlengangs für wenigstens fünf Wellenlängen $\lambda_r$, $\lambda_r$-$\Delta\lambda$, $\lambda_r$-3$\Delta\lambda$, $\lambda_r$+$\Delta\lambda$ und $\lambda_r$+2$\Delta\lambda$ folgende Bedingungen erfüllt:

$$\left|T_b(\lambda_r+\Delta_\lambda) - T_b(\lambda_r+2\Delta\lambda)\right| > 10\%$$

$$\left|T_b(\lambda_r-\Delta\lambda) - T_b(\lambda_r-3\Delta\lambda)\right| < 5\%,\ \text{bevorzugt} <3\%$$

$$T_b(\lambda_r) > 0{,}5\ \%$$

$$T_b(\lambda_r+\Delta\lambda) > 0\ ,\ 5\ \%$$

$$T_b(\lambda_r+2\Delta\lambda) > 0{,}5\ \%$$

$$T_b(\lambda_r-\Delta\lambda) > 0{,}3\ \%$$

$$T_b(\lambda_r-3\Delta\lambda) > 0{,}3\ \%$$

mit

$$4\ nm < \Delta\lambda < 6\ nm.$$

2. Vorrichtung nach Anspruch 1,
   **dadurch gekennzeichnet, daß** die Referenzwellenlänge $\lambda_r$ gleich der Schnittpunktswellenlänge $\lambda_s$ ist.

3. Vorrichtung nach Anspruch 1 oder 2,
   **dadurch gekennzeichnet, daß** sich die beiden Übertragungsfunktionen $T_1(\lambda)$ und $T_b(\lambda)$ bei einem Transmissionswert von weniger als 10%, bevorzugt bei einem Wert von weniger als 5% schneiden.

4. Vorrichtung nach einem der Ansprüche 1 bis 3,
   **dadurch gekennzeichnet, daß** die spektrale Übertragungsfunktion $T_1(\lambda)$ des Beleuchtungsstrahlengangs im Bereich $\lambda_r$...$\lambda_r$+3$\Delta\lambda$ und/oder die spektrale Übertragungsfunktion des Beobachtungsstrahlengangs $T_b(\lambda)$ im Bereich $\lambda_r$...$\lambda_r$-3$\Delta\lambda$ ein nahezu horizontales Plateau aufweist.

5. Vorrichtung nach einem der Ansprüche 1 bis 4,
   **dadurch gekennzeichnet, daß** die spektrale Übertragungsfunktion $T1(\lambda)$ des Beleuchtungsstrahlengangs im Bereich $\lambda_r$...$\lambda_r$+3$\Delta\lambda$ und/oder die spektrale Übertragungsfunktion $T_b(\lambda)$ des Beobachtungsstrahlengangs im Bereich $\lambda_r$...$\lambda_r$-3$\Delta\lambda$ ein lokales Maximum aufweist.

6. Vorrichtung nach einem der Ansprüche 1 bis 5,
   **dadurch gekennzeichnet, daß** der spektrale Übertragungsfunktion $T_1(\lambda)$ des Beleuchtungsstrahlengangs für ALA als Photosensibilisator folgende Beziehung erfüllt

$$100\% > T_1(\lambda=400..420) \geq 80\%$$

$$15\% \geq T_1(\lambda=440..455) \geq 0,5\%$$

**7.** Vorrichtung nach einem der Ansprüche 1 bis 6,
**dadurch gekennzeichnet, daß** die spektrale Übertragungsfunktion der lichtübertragenden und der bildgebenden Einheit mittels eines oder mehrerer optischer Elemente einstellbar sind.

**8.** Vorrichtung nach Anspruch 7,
**dadurch gekennzeichnet, daß** die optischen Elemente Interferenzfilter sind.

**9.** Vorrichtung nach einem der Ansprüche 1 bis 8,
**dadurch gekennzeichnet, daß** die spektralen Übertragungsfunktionen derart eingestellt sind, daß die Gesamtintensität des induzierten Fluoreszenzlichtes in der gleichen Größenordnung wie die Gesamtintensität des direkt an dem Gewebebereich reflektierten Anteils des Lichts des Beleuchtungssystems liegt.

**Claims**

**1.** Endoscopic or microscopic device for an "in vivo" diagnosis by means of a reaction which is light-induced by a photosensitizer or evoked by autofluorescence in biological tissue, comprising

- an illuminating beam path, formed by

    an illuminating system having at least one light source (6) with a lamp system generating incoherent light in a wavelength range of at least 400 to 650 nm, and
    a light guiding unit (9, 5) which directs the light of the illuminating system onto the tissue region to be diagnosed or therapeutically treated,

    and having a spectral transmission function $T_1(\lambda)$ matched to the fluorescence excitation spectrum of the photosensitizer or the tissue; and
- an observation beam path, formed by

    an imaging unit (4), an image recording unit (8), and if needed, an image transmitting unit which images the light issuing from the tissue region onto an image plane,

    and having a spectral transmission function $T_b(\lambda)$ matched to the fluorescence spectrum of the photosensitizer or the tissue,

wherein the spectral transmission function $T_1(\lambda)$ of the illuminating beam path and the spectral transmission function $T_b(\lambda)$ of the observation beam path intersect at a wavelength $\lambda_s$ at which the transmission value of each beam path is no higher than 30%,
**characterized in that** there is at least one reference wavelength $\lambda_r$ that is at most $2\Delta\lambda$ larger or smaller than the intersection-point wavelength $\lambda_s$, i.e. for which

$$\lambda_s - 2\Delta\lambda < \lambda_r < \lambda_s + 2\Delta\lambda$$

and based on which

- the spectral transmission function $T_1(\lambda)$ of the illuminating beam path satisfies the following conditions for at least five wavelengths $\lambda_r$, $\lambda_r + \Delta\lambda$, $\lambda_r + 3\Delta\lambda$, $\lambda_r - \Delta\lambda$, and $\lambda_r - 2\Delta\lambda$:

$$\left| T_1(\lambda_r - \Delta\lambda) - T_1(\lambda_r - 2\Delta\lambda) \right| > 10\%$$

$$\left|T_1\ (\lambda_r + \Delta\lambda) - T_1\ (\lambda_r + 3\Delta\lambda)\right| < 5\%, \text{ preferably} < 3\%$$

$$T_1(\lambda_r) > 0.5\%$$

$$T_1\ (\lambda_r - \Delta\lambda) > 0.5\%$$

$$T_1(\lambda_r - 2\Delta\lambda) > 0.5\%$$

$$T_1\ (\lambda_r + \Delta\lambda) > 0.3\%$$

$$T_1\ (\lambda_r + 3\Delta\lambda) > 0.3\%$$

wherein

$$4\ nm < \Delta\lambda < 6\ nm,$$

- and the spectral transmission function $T_b(\lambda)$ of the observation beam path satisfies the following conditions for at least five wavelengths $\lambda_r$, $\lambda_r - \Delta\lambda$, $\lambda_r - 3\Delta\lambda$, $\lambda_r + \Delta\lambda$, and $\lambda_r + 2\Delta\lambda$:

$$\left|T_b\ (\lambda_r + \Delta\lambda) - T_b\ (\lambda_r + 2\Delta\lambda)\right| > 10\%$$

$$\left|T_b\ (\lambda_r - \Delta\lambda) - T_b\ (\lambda_r - 3\Delta\lambda)\right| < 5\%, \text{ preferably} < 3\%$$

$$T_b(\lambda_r) > 0.5\%$$

$$T_b(\lambda_r + \Delta\lambda) > 0.5\%$$

$$T_b(\lambda_r + 2\Delta\lambda) > 0.5\%$$

$$T_b(\lambda_r - \Delta\lambda) > 0.3\%$$

$$T_b\ (\lambda_r - 3\Delta\lambda) > 0.3\%$$

wherein

$$4\ nm < \Delta\lambda < 6\ nm.$$

**2.** Device according to claim 1,
**characterized in that** the reference wavelength $\lambda_r$ is equal to the intersection-point wavelength $\lambda_s$.

**3.** Device according to claim 1 or 2,
**characterized in that** the two transmission functions $T_1(\lambda)$ and $T_b(\lambda)$ intersect at a transmission value of less than 10%, preferably at a value of less than 5%.

4. Device according to any one of claims 1 to 3,
**characterized in that** the spectral transmission function $T_1(\lambda)$ of the illuminating beam path has an almost horizontal plateau in the range $\lambda_r \ldots \lambda_r + 3\Delta\lambda$, and/or the spectral transmission function $T_b(\lambda)$ of the observation beam path has an almost horizontal plateau in the range $\lambda_r \ldots \lambda_r - 3\Delta\lambda$.

5. Device according to any one of claims 1 to 4,
**characterized in that** the spectral transmission function $T_1(\lambda)$ of the illuminating beam path has a local maximum in the range $\lambda_r \ldots \lambda_r + 3\Delta\lambda$, and/or the spectral transmission function $T_b(\lambda)$ of the observation beam path has a local maximum in the range $\lambda_r \ldots \lambda_r - 3\Delta\lambda$.

6. Device acccording to any one of claims 1 to 5,
**characterized in that** the spectral transmission function $T_1(\lambda)$ of the illuminating beam path satisfies the following relationship with ALA as a photosensitizer:

$$100\% > T_1(\lambda = 400 .. 420) \geq 80\%$$

$$15\% \geq T_1(\lambda = 440 .. 455) \geq 0.5\%.$$

7. Device according to any one of claims 1 to 6,
**characterized in that** the spectral transmission function of the light transmitting unit and the imaging unit can be set by means of one or a plurality of optical elements.

8. Device according to claim 7,
**characterized in that** the optical elements are interference filters.

9. Device according to any one of claims 1 to 8,
**characterized in that** the spectral transmission functions are set in such manner that the total intensity of the induced fluorescence light is of the same order of magnitude as the total intensity of the proportion of the light of the illuminating system directly reflected from the tissue region.

**Revendications**

1. Dispositif endoscopique ou microscopique destiné au diagnostic par une réaction induite par la lumière au moyen d'un photosensibilisateur ou provoquée par une fluorescence propre *in vivo* dans un tissu biologique, avec

   - un faisceau d'éclairage formé par

      un système d'éclairage qui présente au moins une source de lumière (6) avec un système de lampe produisant de la lumière incohérente dans une plage de longueur d'onde d'au moins 400 à 650 nm, et un ensemble guide de lumière (9, 5) qui dirige la lumière du système d'éclairage sur la zone du tissu à diagnostiquer et/ou à traiter,
      qui présente une fonction de transmission spectrale $T_1(\lambda)$ qui est adaptée au spectre d'excitation de la fluorescence du photosensibilisateur ou du tissu, et

   - un faisceau d'observation formé par

      un ensemble de formation d'image (4), un ensemble de saisie d'image (8) ainsi le cas échéant qu'un ensemble de transmission d'image qui transforme la lumière entrant dans la zone du tissu en un plan image,
      qui présente une fonction de transmission spectrale $T_b(\lambda)$ qui est adaptée au spectre de fluorescence du photosensibilisateur ou du tissu,

   dans lequel la fonction de transmission spectrale $T_1(\lambda)$ du faisceau d'éclairage et la fonction de transmission spectrale $T_b(\lambda)$ du faisceau d'observation se coupent à une longueur d'onde $\lambda_s$ à laquelle le coefficient de transmission de chaque faisceau n'est pas supérieur à 30%,

**caractérisé en ce qu'**il fournit au moins une longueur d'onde de référence $\lambda_r$ qui est supérieure ou inférieure d'un maximum de $2\Delta\lambda$ à la longueur d'onde au point d'intersection $\lambda_s$, c'est-à-dire pour laquelle on a :

$$\lambda_s - 2\Delta\lambda < \lambda_r < \lambda_s + 2\Delta\lambda$$

et à partir de laquelle

- la fonction de transmission spectrale $T_1(\lambda)$ du faisceau d'éclairage répond pour au moins cinq longueurs d'ondes $\lambda_r$, $\lambda_r + \Delta\lambda$, $\lambda_r + 3\Delta\lambda$, $\lambda_r - \Delta\lambda$ et $\lambda_r - 2\Delta\lambda$ aux conditions suivantes :

$$\left|T_1(\lambda_r - \Delta\lambda) - T_1(\lambda_r - 2\Delta\lambda)\right| > 10\%$$

$$\left|T_1(\lambda_r + \Delta\lambda) - T_1(\lambda_r + 3\Delta\lambda)\right| < 5\%,$$

de préférence < 3%

$$T_1(\Delta\lambda_r) > 0,5\%$$

$$T_1(\lambda_r - \Delta\lambda) > 0,5\%$$

$$T_1(\lambda_r - 2\Delta\lambda) > 0,5\%$$

$$T_1(\lambda_r + \Delta\lambda) > 0,3\%$$

$$T_1(\lambda_r + 3\Delta\lambda) > 0,3\%$$

avec

$$4 \text{ nm} < \Delta\lambda < 6 \text{ nm},$$

- et la fonction de transmission spectrale $T_b(\lambda)$ du faisceau d'observation répond pour au moins cinq longueurs d'ondes $\lambda_r$, $\lambda_r - \Delta\lambda$, $\lambda_r - 3\Delta\lambda$, $\lambda_r + \Delta\lambda$ et $\lambda_r + 2\Delta\lambda$ aux conditions suivantes :

$$\left|T_b(\lambda_r + \Delta\lambda) - T_b(\lambda_r + 2\Delta\lambda)\right| > 10\%$$

$$\left|T_b(\lambda_r - \Delta\lambda) - T_b(\lambda_r - 3\Delta\lambda)\right| < 5\%,$$

de préférence < 3%

$$T_b(\lambda_r) > 0,5\%$$

$$T_b(\lambda_r + \Delta\lambda) > 0,5\%$$

$$T_b(\lambda_r + 2\Delta\lambda) > 0,5\%$$

$$T_b(\lambda_r - \Delta\lambda) > 0{,}3\%$$

$$T_b(\lambda_r - 3\Delta\lambda) > 0{,}3\%$$

avec

$$4 \text{ nm} < \Delta\lambda < 6 \text{ nm,}$$

2. Dispositif selon la revendication 1, **caractérisé en ce que** la longueur d'onde de référence $\lambda_r$ est égale à la longueur d'onde au point d'intersection $\lambda_s$.

3. Dispositif selon la revendication 1 ou la revendication 2, **caractérisé en ce que** les deux fonctions de transmission $T_1(\lambda)$ et $T_b(\lambda)$ se coupent à un coefficient de transmission inférieur à 10%, de préférence à un coefficient inférieur à 5%.

4. Dispositif selon l'une des revendications 1 à 3, **caractérisé en ce que** la fonction de transmission spectrale $T_1(\lambda)$ du faisceau d'éclairage présente un palier pratiquement horizontal dans la plage de $\lambda_r \ldots \lambda_r + 3\Delta\lambda$ et/ou la fonction de transmission spectrale $T_b(\lambda)$ du faisceau d'observation présente un palier pratiquement horizontal dans la plage de $\lambda_r \ldots \lambda_r - 3\Delta\lambda$.

5. Dispositif selon l'une des revendications 1 à 4, **caractérisé en ce que** la fonction de transmission spectrale $T_1(\lambda)$ du faisceau d'éclairage présente un maximum local dans la plage de $\lambda_r \ldots \lambda_r + 3\Delta\lambda$ et/ou la fonction de transmission spectrale $T_b(\lambda)$ du faisceau d'observation présente un maximum local dans la plage de $\lambda_r \ldots \lambda_r - 3\Delta\lambda$.

6. Dispositif selon l'une des revendications 1 à 5, **caractérisé en ce que**, lorsque l'ALA est utilisée comme photosensibilisateur, la fonction de transmission spectrale $T_1(\lambda)$ du faisceau d'éclairage répond à la relation suivante :

$$100\% > T_1(\lambda = 400 \ .. \ 420) \geq 80\%$$

$$15\% \geq T_1\ (\lambda = 440..455) \geq 0{,}5\%$$

7. Dispositif selon l'une des revendications 1 à 6, **caractérisé en ce que** les fonctions de transmission spectrale de l'ensemble de transmission de lumière et de l'ensemble de formation d'image sont réglables au moyen d'un ou plusieurs éléments optiques.

8. Dispositif selon la revendication 7, **caractérisé en ce que** les éléments optiques sont des filtres interférentiels.

9. Dispositif selon l'une des revendications 1 à 8, **caractérisé en ce que** les fonctions de transmission spectrale sont ajustées de telle façon que l'intensité totale de la lumière fluorescente induite est du même ordre de grandeur que l'intensité totale de la partie de la lumière du système d'éclairage qui est directement réfléchie au niveau de la zone de tissu.

FIG 1

# FIG 2

EP 0 930 843 B1

## FIG 3

EP 0 930 843 B1

# FIG 4A

EP 0 930 843 B1

# FIG 4B

EP 0 930 843 B1